# EUROPÄISCHE PATENTSCHRIFT

(11) **EP 3 417 761 B1**
(45) Veröffentlichungstag und Bekanntmachung des Hinweises auf die Patenterteilung: **08.04.2020**
(21) Anmeldenummer: 18176476.2
(22) Anmeldetag: 07.06.2018
(51) Int. Cl.: A61B 1/002, G02B 1/11, G02B 5/00, G02B 23/24

(54) **RELAISOPTIK FÜR EIN STARRES ENDOSKOP UND EIN ENDOSKOP MIT EINER SOLCHEN RELAISOPTIK**
RELAY OPTIC FOR A RIGID ENDOSCOPE AND AN ENDOSCOPE WITH SUCH A RELAY OPTIC
OPTIQUE DE RELAIS POUR UN ENDOSCOPE RIGIDE ET UN ENDOSCOPE AVEC UNE TELLE OPTIQUE DE RELAIS

(30) Priorität: 16.06.2017 DE 102017113271
(43) Veröffentlichungstag der Anmeldung: 26.12.2018
(73) Patentinhaber: avateramedical GmbH, 07745 Jena (DE)
(72) Erfinder: KHETTAL, Ali, 12435 Berlin (DE); WEISE, Fabian, 10437 Berlin (DE)
(74) Vertreter: Schaumburg und Partner Patentanwälte mbB

(56) Entgegenhaltungen:
- EP-A1- 3 229 056
- DE-A1-102015 119 622
- US-A- 5 684 629
- US-A- 5 805 345
- US-B1- 6 490 085

## Beschreibung

Die Erfindung betrifft eine Relaisoptik für ein starres Endoskop, die zwei identisch ausgebildete Linsensysteme enthält, die bezüglich einer senkrecht zur optischen Achse liegenden Symmetrieebene symmetrisch zueinander angeordnet sind. Ferner betrifft die Erfindung ein Endoskop, das ein Relaissystem mit mindestens einer solchen Relaisoptik umfasst.

Endoskope werden insbesondere in der minimalinvasiven Chirurgie verwendet, um dem operierenden Arzt Einblick in die Körperregion zu gewähren, in der das Operationsgebiet liegt. Verwendet werden sowohl monokulare Endoskope als auch stereoskopische Endoskope, wobei letztere über zwei optische Kanäle einen räumlichen Eindruck der Tiefe liefern, was mit monokularen Endoskopen nicht möglich ist.

Am distalen Ende eines Endoskopschafts ist typischerweise ein Objektiv angeordnet, welches das vom zu beobachtenden Objekt ausgehende Licht sammelt und ein reelles Zwischenbild des Objektes erzeugt. Dieses Zwischenbild wird mit Hilfe eines dem Objektiv nachgeordneten optischen Relaissystems zum proximalen Ende des Endoskopschafts übertragen. Am proximalen Ende des Endoskopschafts ist ein Okular angeordnet, welches das reelle Zwischenbild entweder für das menschliche Auge oder mittels eines Kameraobjektivs auf eine Sensoroberfläche abbildet.

Ein optisches Relaissystem zur Verwendung in einem Endoskop sollte dabei eine hohe optische Qualität bei geringem Durchmesser aufweisen. Während in flexiblen Endoskopen zu diesem Zweck häufig Lichtleiter, z.B. Bündel aus Glasfasern, verwendet werden, haben sich zur Verwendung in starren Endoskopen Relaissysteme mit Stablinsen durchgesetzt. Insbesondere aus speziellen optischen Gläsern gefertigte Stablinsen besitzen eine höhere optische Qualität als flexible Lichtleiter.

In dem Dokument US 5 557 454 A ist ein starres Endoskop offenbart, welches mit Hilfe von Stablinsen den optischen Bildtransport von einem distal in dem Endoskopschaft angebrachten Objektiv zu einem proximal angeordneten Okular realisiert.

Aus dem Dokument US 6,490,085 B1 ist ein optisches Relaissystem für ein starres Endoskop bekannt, welches zwei Linsensysteme enthält, die symmetrisch bezüglich einer senkrecht zur optischen Achse liegenden Symmetrieebene angeordnet sind.

In dem Dokument DE 10 2013 209 956 A1 ist ein starres stereoskopisches Endoskop offenbart, bei dem der Bildtransport von einem proximal angebrachten Objektiv zu einer distal angebrachten Sensoroberfläche wiederum mithilfe von Stablinsen erfolgt

In dem Dokument WO 2015/131278 A1 ist ein für den Bildtransport zur Breitbandbildgebung vorgesehenes System beschrieben, das aus spiegelsymmetrisch angeordneten Stablinsen und sphärischen Linsen besteht.

EP 3 229 056 A1 offenbart ein Optiksystem für ein starres Endoskop. Das Optiksystem umfasst mehrere Paare aus jeweils identisch ausgebildeten Linsensystemen. Die Linsensysteme sind jeweils bezüglich einer zur optischen Achse senkrecht liegenden Ebene symmetrisch zueinander angeordnet. Jedes Linsensystem umfasst eine erste bikonvexe Linse, eine bikonkave Linse, eine Stablinse und eine zweite bikonvexe Linse. Dieses am 11.10.2017 veröffentlichte Dokument fällt unter Artikel 54 (3) EPÜ.

US 5 684 629 A offenbart ein Relaislinsensystem für ein Endoskop. Das Relaislinsensystem umfasst wenigstens ein Relaislinsenmodul mit zwei identischen Linsensystemen, die jeweils bezüglich einer zur optischen Achse senkrecht liegenden Ebene symmetrisch zueinander angeordnet sind.

Einige der aus dem Stand der Technik bekannten optischen Relaissysteme korrigieren Abbildungsfehler entweder gar nicht oder jedenfalls nicht ausreichend. Insbesondere weisen einige der bekannten optischen Relaissysteme eine große Bildfeldwölbung auf, die sich mit nachfolgenden Optiksystemen nur schwierig korrigieren lässt. Bekannte optische Relaissysteme sind zudem nur auf eine bestimmte Endoskopbaulänge hin ausgelegt.

Die Aufgabe der Erfindung besteht darin, eine Relaisoptik für ein starres Endoskop anzugeben, die einfach und kompakt ausgebildet ist und gleichzeitig Bildfehler, insbesondere die Bildfeldwölbung, weitestgehend korrigiert.

Diese Aufgabe wird durch eine Relaisoptik mit den Merkmalen des Anspruchs 1 und ein Endoskop mit den Merkmalen des Anspruchs 6 gelöst. Vorteilhafte Weiterbildungen sind in den abhängigen Ansprüchen angegeben.

Die erfindungsgemäße Relaisoptik umfasst zwei identisch ausgebildete Linsensysteme, die bezüglich einer senkrecht zur optischen Achse liegenden Symmetrieebene symmetrisch zueinander angeordnet sind. Die Linsensysteme enthalten jeweils eine erste bikonvexe Linse, eine bikonkave Linse, eine Stablinse, die eine der Symmetrieebene zugewandte konvexe Linsenfläche und eine von der Symmetrieebene abgewandte konkave Linsenfläche hat, und eine zweite bikonvexe Linse in dieser Reihenfolge von der Symmetrieebene her gesehen, wobei die erste bikonvexe Linse und/oder die zweite bikonvexe Linse des jeweiligen Linsensystems eine Antireflexionsschicht aufweisen. Hierdurch wird ein einfacher und kompakter Aufbau der Relaisoptik erreicht.

Durch die symmetrische (spiegelbildliche) Anordnung der identisch ausgebildeten Linsensysteme ist es möglich, die chromatische Aberration, die in einem Linsensystem auftritt, durch das andere Linsensystem weitestgehend zu korrigieren. Die Korrektur der chromatischen Aberration ermöglicht es insbesondere, mehrere Relaisoptiken innerhalb eines Relaissystems hintereinander anzuordnen, ohne dass die chromatische Gesamtaberration des Relaissystems zu groß wird.

Die erfindungsgemäße Relaisoptik hat ferner den Vorteil eines vergleichsweise geringen Herstellungs- und Montageaufwands durch die Verwendung lediglich weniger unterschiedlicher Bauteile.

Die spezielle Ausgestaltung der Relaisoptik ermöglicht es auch, dass eine Bildfeldwölbung, die beispielsweise durch ein der Relaisoptik vorgeordnetes Objektiv verursacht wird, durch die Relaisoptik selbst korrigiert wird. Insbesondere in einem Relaissystem, das eine beliebige Anzahl hintereinander angeordneter Relaisoptiken enthält, kann so die Bildfeldwölbung fast vollständig korrigiert werden. Ferner lässt sich durch die Korrektur der Bildfeldwölbung in der erfindungsgemäßen Relaisoptik ein bildseitig der Relaisoptik angeordnetes Okular kompakter als in bisher bekannten Endoskopen konstruieren, da vorliegend die Bildfeldwölbung in der Relaisoptik selbst und nicht wie üblich im Okular korrigiert wird.

In einer vorteilhaften Weiterbildung sind die erste bikonvexe Linse, die bikonkave Linse, die Stablinse und/oder die zweite bikonvexe Linse des jeweiligen Linsensystems miteinander verkittet. Besonders vorteilhaft ist es, alle vorgenannten Linsen zu einem einzigen Bauteil zu verkitten. Dieses Bauteil wird im Folgenden auch als verkittete Staboptik bezeichnet. Durch die Verwendung verkitteter Staboptiken lässt sich der Herstellungs- und Montageaufwand erheblich verringern.

In einer weiteren vorteilhaften Weiterbildung besteht die Stablinse des jeweiligen Linsensystems aus einem Kronglas. Die Verwendung insbesondere von Barium-Kronglas ist auf Grund seiner guten optischen Übertragungseigenschaften vorteilhaft für eine Relaisoptik.

Vorzugsweise bestehen die erste bikonvexe Linse, die bikonkave Linse und/oder die zweite bikonvexe Linse des jeweiligen Linsensystems aus Flintglas. Die hohe Dispersion von Flintgläsern erlaubt die Konstruktion von verkitteten Staboptiken mit gewünschten achromatischen Eigenschaften, insbesondere durch die Kombination leichter und schwerer Flintgläser, die unterschiedliche Abbe- und Brechzahlen haben.

Die erste bikonvexe Linse und/oder die zweite bikonvexe Linse des jeweiligen Linsensystems weisen eine Antireflexionsschicht, insbesondere auf ihrer unverkitteten Fläche, auf. Dies dient insbesondere der Erhöhung der optischen Transmission sowie der Minimierung von Streulicht und somit der Verbesserung der optischen Qualität der Relaisoptik. Die Erhöhung der optischen Transmission erlaubt es, mehrere Relaisoptiken ohne signifikanten Lichtverlust hintereinander zu schalten.

In einer weiteren vorteilhaften Ausgestaltung ist eine Blende in der Symmetrieebene angeordnet, um die Apertur der Relaisoptik zu begrenzen.

Ein weiterer Aspekt der Erfindung betrifft ein Endoskop, das ein Relaissystem mit mindestens einer Relaisoptik nach oben beschriebener Art umfasst.

In einer vorteilhaften Ausgestaltung umfasst das Endoskop einen starren Endoskopschaft, in dem das Relaissystem angeordnet ist.

In einer besonders vorteilhaften Ausgestaltung umfasst das Relaissystem mindestens ein Relaismodul, das mehrere längs der optischen Achse hintereinander angeordnete Relaisoptiken nach oben beschriebener Art enthält.

In einer weiteren vorteilhaften Ausgestaltung umfasst das Relaissystem zwei Relaismodule, die eine stereoskopische Anordnung bilden. Somit sind mit Hilfe des Endoskops stereoskopische Beobachtungen möglich.

Das erfindungsgemäße optische Design umfasst insbesondere eine Kombination von Linsenelementen und einer Stablinse innerhalb einer Kittgruppe (Stablinsensystem) und eine Kombination zweier Stablinsensysteme zu einer Relaisoptik. Die Radien und Materialien der Einzellinsen sind vorzugsweise so aufeinander abgestimmt, dass die sogenannten Seidelschen Abbildungsfehler besonders gut korrigiert werden. Dabei wird insbesondere die Petzval-Summe als Maß für die Bildfeldwölbung minimiert. Durch die Verwendung zweier identischer Stablinsensysteme in umgekehrter Reihenfolge kann zudem die chromatische Aberration des ersten Stablinsensystems durch das zweite Stablinsensystem kompensiert werden. Weiterhin können nicht nur die Zwischenbilder, welche optisch eine Relaisoptik begrenzen, besonders gut korrigiert werden, sondern es kann auch die Pupillenabbildung, welche von einer Relaisoptik in die nächste Relaisoptik überführt, korrigiert werden.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: eine Relaisoptik für ein starres Endoskop gemäß einem Ausführungsbeispiel;
- Figur 2: ein Ausführungsbeispiel eines Linsensystems, das Teil der Relaisoptik nach Figur 1 ist;
- Figur 3: ein Ausführungsbeispiel eines monokularen Endoskops, das mehrere Relaisoptiken nach Figur 1 enthält; und
- Figur 4: ein Ausführungsbeispiel eines stereoskopischen Endoskops, das mehrere Relaisoptiken nach Figur 1 enthält.

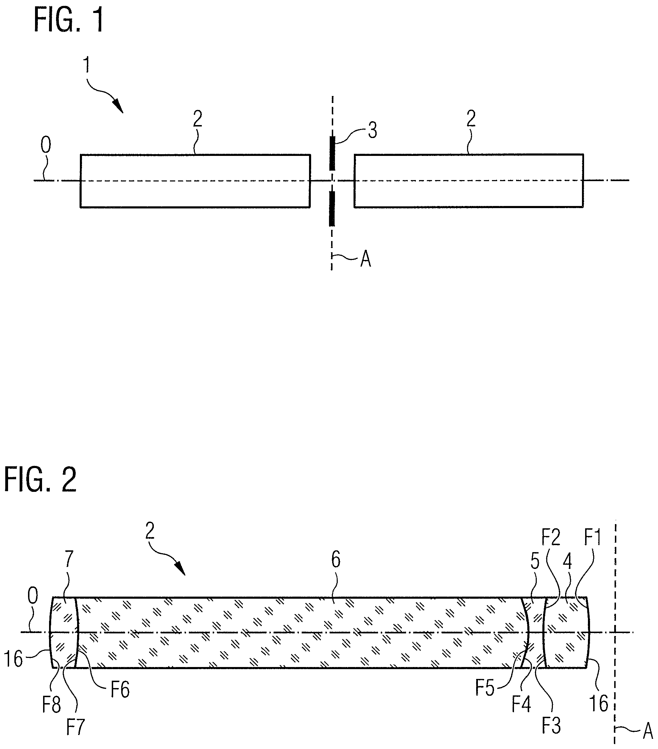

Figur 1 zeigt ein Ausführungsbeispiel einer Relaisoptik 1 zur Verwendung in einem starren Endoskop. Die Relaisoptik 1 umfasst zwei identisch ausgebildete Linsensysteme 2 und eine Blende 3, die zwischen den Linsensystemen 2 in einer Ebene A angeordnet ist, die senkrecht auf der optischen Achse O des Endoskops steht. Beide Linsensysteme 2 der Relaisoptik 1 sind in Bezug auf die Ebene A spiegelsymmetrisch zueinander ausgebildet.

In Figur 2 ist eines der beiden identisch ausgebildeten Linsensysteme 2 nach Figur 1 schematisch dargestellt. Das Linsensystem 2 umfasst von der Ebene A her gesehen eine erste bikonvexe Linse 4, eine bikonkave Linse 5, eine Stablinse 6 und eine zweite bikonvexe Linse 7. Die erste bikonvexe Linse 4 weist zwei konvexe Flächen F1, F2 auf. Die bikonkave Linse 5 weist zwei konkave Flächen F3, F4 auf. Die Stablinse 6 weist eine der Ebene A zugewandte konvexe Fläche F5 und eine der Ebene A abgewandte konvexe Fläche F6 auf. Die zweite bikonvexe Linse 7 weist zwei konvexe Flächen F7, F8 auf.

Die der Ebene A abgewandte Fläche F2 der ersten bikonvexen Linse 4 ist mit der der Ebene A zugewandten Fläche F3 der bikonkaven Linse 5 zu einer einzigen optisch wirksamen Fläche verkittet. Die der Ebene A abgewandte Fläche F4 der bikonkaven Linse 5 ist mit der der Ebene A zugewandten Fläche F5 der Stablinse 6 verkittet. Die der Ebene A abgewandte Fläche F6 der Stablinse 6 ist mit der der Ebene A zugewandten Fläche F7 der zweiten bikonvexen Linse 7 verkittet. Somit bildet das Linsensystem 2 ein einziges Bauteil.

Die unverkitteten Linsenflächen F1, F8 der ersten bikonvexen Linse 4 bzw. der zweiten bikonvexen Linse 7 weisen ferner jeweils eine Antireflexionsschicht 16 auf. Diese dienen der Verminderung von Streulicht.

Tabelle 1 zeigt die Linsendaten der Relaisoptik 1 nach Figur 1 mit den beiden identischen, symmetrisch um die Blende 3 angeordneten Linsensystemen 2 nach Figur 2. Die optisch wirksamen Flächen der Relaisoptik 1 sind in Tabelle 1 von der Objektseite her mit 1 bis 10 durchnummeriert. Die dazugehörigen Bezugszeichen nach Figur 2 sind in Klammern angegeben. Alle Dimensionen bzw. Längenangaben sind in der Einheit [mm]. Die Bezeichnung der Gläser folgt der Nomenklatur von Schott.

**Tabelle 1**

| Fläche | Radius | Dicke | Glas | Durchmesser |
|---|---|---|---|---|
| Objekt | Unendlich | 0 | | 2,5 |
| Aperturblende | Unendlich | 4,40117 | | 2,5 |
| 1 (F8) | 25,18 | 1,3 | N-LASF41 | 3,6 |
| 2 (F6, F7) | -14,3 | 23,7 | N-BAK1 | 3,6 |
| 3 (F4, F5) | -4,475 | 0,8 | N-KZFS11 | 3,6 |
| 4 (F2, F3) | 13,455 | 2,3 | N-LASF44 | 3,6 |
| 5 (F1) | -18,265 | 1,979656 | | 3,6 |
| Blende (3) | Unendlich | 1,979656 | | 3,6 |
| 6 (F1) | 18,265 | 2,3 | N-LASF44 | 3,6 |
| 7 (F2, F3) | -13,455 | 0,8 | N-KZFS11 | 3,6 |
| 8 (F4, F5) | 4,475 | 23,7 | N-BAK1 | 3,6 |
| 9 (F6, F7) | 14,3 | 1,3 | N-LASF41 | 3,6 |
| 10 (F8) | -25,18 | 4,40117 | | 3,6 |
| Bild | -12,5 | | | 2,513237 |

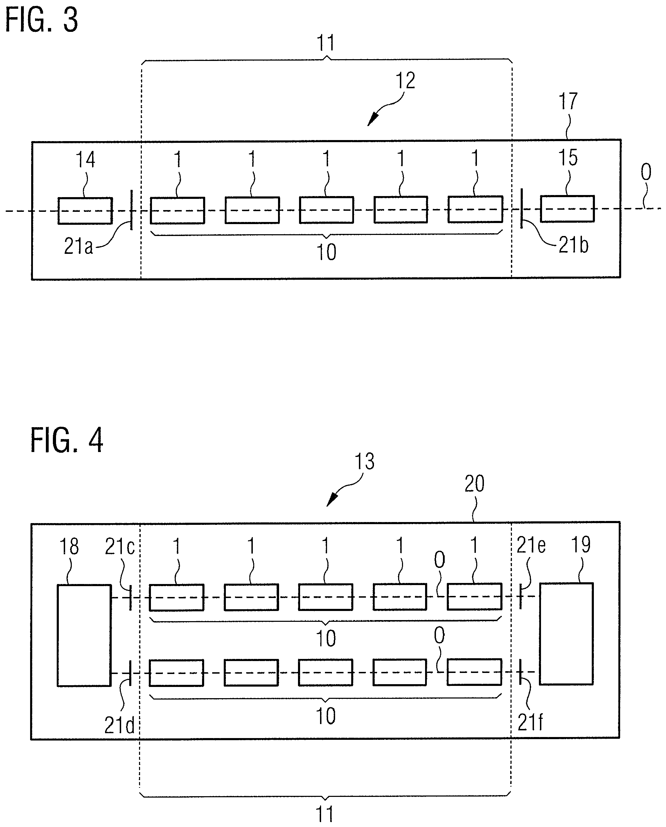

Eine mögliche Ausführungsform eines monokularen Endoskops 12 ist schematisch in Figur 3 dargestellt. Es umfasst ein distal angeordnetes Objektiv 14, ein optisches Relaissystem 11 mit einem Relaismodul 10 und ein proximal angeordnetes Okular 15. Das Endoskop 12 umfasst ferner einen Schaft 17, in dem die vorgenannten Bauteile bzw. optischen Elemente 10, 14 und 15 angeordnet sind. Das Relaismodul 10 enthält mehrere längs der optischen Achse O hintereinander angeordnete Relaisoptiken 1 nach Figur 1.

Die Funktionsweise des in Figur 3 gezeigten Endoskops 12 ist insbesondere derart, dass das am distalen Ende des Endoskops 12 angeordnete Objektiv 14 ein erstes Zwischenbild 21a des zu beobachtenden Objektes erzeugt. Das Relaismodul 10 bildet das distale, erste Zwischenbild 21a auf ein proximales, zweites Zwischenbild 21b ab. Damit überträgt das Relaissystem 11 bzw. das Relaismodul 10 das erste Zwischenbild 21a gleichsam vom distalen zum proximalen Ende des Endoskops 12. Das am proximalen Ende des Endoskops 12 angeordnete Okular 15 bildet schließlich das zweite Zwischenbild 21b auf einen in Figur 3 nicht gezeigten Kamerasensor ab.

Die jeweils zwei identische Linsensysteme 2 nach Figur 2 umfassenden Relaisoptiken 1 des Relaismoduls 10 sind insbesondere in Bezug auf die chromatische Aberration selbstkorrigierend. D.h. jede der Relaisoptiken 1 ist für sich betrachtet hinsichtlich der chromatischen Aberration nahezu vollständig korrigiert. Dies erlaubt es, die Relaisoptiken 1 in Vielzahl innerhalb des Relaismoduls 10 hintereinander anzuordnen, ohne dass die chromatische Gesamtaberration des Relaissystems 11 zu groß wird. Dadurch kann das Endoskop 12 in unterschiedlichen Baulängen bei im Wesentlichen gleicher optischer Qualität realisiert werden.

Ferner muss die Korrektion der Abbildungsfehler auch nicht durch das dem Relaismodul 10 nachgeordnete Okular 15 erfolgen. Das Okular 15 kann deswegen besonders kompakt ausgeführt werden.

Die einzelnen Relaisoptiken 1 des Relaissystems 11 bzw. des Relaismoduls 10 bilden jeweils ein optisches Umkehrsystem mit Abbildungsmaßstab -1. Da die Relaisoptiken 1 in einer ungeraden Anzahl (z.B. fünf) in dem Relaismodul 10 angeordnet sind, bildet das Relaissystem 11 ein optisches System mit Abbildungsmaßstab +1.

Ein Ausführungsbeispiel eines stereoskopischen Endoskops 13 ist in Figur 4 schematisch dargestellt. Gegenüber dem in Figur 3 dargestellten monokularen Endoskop 12 weist das stereoskopische Endoskop 13 zwei optische Kanäle auf. Das stereoskopische Endoskop 13 hat einen Schaft 20, in dem vom distalen Ende her betrachtet ein Objektiv 18, ein stereoskopisches Relaissystem 11 mit zwei Relaismodulen 10 und ein proximal angeordnetes Okular 19 angeordnet sind.

In dem stereoskopischen Relaissystem 11 ist jeweils eines der beiden Relaismodule 10 einem der beiden optischen Kanäle zugeordnet. Jedes der beiden Relaismodule 10 bildet ein distales Zwischenbild 21c bzw. 21d, das durch das Objektiv 18 erzeugt wird, auf ein zweites proximales Zwischenbild 21e bzw. 21f ab. Die dadurch erzeugten proximalen Zwischenbilder 21e bzw. 21f werden dann durch das Okular 19 auf einen in Figur 4 nicht gezeigten Kamerasensor abgebildet.

Die vorgenannten Ausführungsformen nach den Figuren 3 und 4 sind beispielhaft zu verstehen. So ist insbesondere die Zahl der Relaisoptiken 1 weder auf eine ungerade Zahl noch konkret auf die Zahl fünf beschränkt.

Das stereoskopische Endoskop 13 nach Figur 4 sieht für die beiden optischen Kanäle ein gemeinsam genutztes Objektiv 18 vor. In einer alternativen Ausführungsform kann jedem einzelnen Kanal ein eigenes Objektiv zugeordnet sein.

### Bezugszeichenliste

- 1: Relaisoptik
- 2: Linsensystem
- 3: Blende
- 4: erste bikonvexe Linse
- 5: bikonkave Linse
- 6: Stablinse
- 7: zweite bikonvexe Linse
- 10: Relaismodul
- 11: Relaissystem
- 12: monokulares Endoskop
- 13: stereoskopisches Endoskop
- 14, 18: Objektiv
- 15, 19: Okular
- 16: Antireflexionsbeschichtung
- 17, 20: Endoskopschaft
- 21a bis 21e: Zwischenbild
- A: Symmetrieebene
- O: optische Achse

## Patentansprüche

1. Relaisoptik (1) für ein starres Endoskop (12, 13), umfassend zwei identisch ausgebildete Linsensysteme (2), die bezüglich einer senkrecht zur optischen Achse (O) liegenden Symmetrieebene (A) symmetrisch zueinander angeordnet sind, wobei mindestens eine Linse des jeweiligen Linsensystems eine Antireflexionsschicht aufweist, **dadurch gekennzeichnet, dass** die Linsensysteme (2) jeweils eine erste bikonvexe Linse (4), eine bikonkave Linse (5), eine Stablinse (6), die eine der Symmetrieebene (A) zugewandte konvexe Linsenfläche (F5) und eine von der Symmetrieebene (A) abgewandte konkave Linsenfläche (F6) hat, und eine zweite bikonvexe Linse (7) in dieser Reihenfolge von der Symmetrieebene (A) her gesehen enthalten, wobei die erste bikonvexe Linse (4) und/oder die zweite bikonvexe Linse (7) des jeweiligen Linsensystems (2) die Antireflexionsschicht aufweisen.

2. Relaisoptik (1) nach Anspruch 1, **dadurch gekennzeichnet, dass** die erste bikonvexe Linse (4), die bikonkave Linse (5), die Stablinse (6) und/oder die zweite bikonvexe Linse (7) des jeweiligen Linsensystems (2) miteinander verkittet sind.

3. Relaisoptik (1) nach Anspruch 1 oder 2, **dadurch gekennzeichnet, dass** die Stablinse (6) des jeweiligen Linsensystems (2) aus einem Kronglas besteht.

4. Relaisoptik (1) nach einem der vorhergehenden Ansprüche, **dadurch gekennzeichnet, dass** die erste bikonvexe Linse (4), die bikonkave Linse (5) und/oder die zweite bikonvexe Linse (7) des jeweiligen Linsensystems (2) aus einem Flintglas bestehen.

5. Relaisoptik (1) nach einem der vorhergehenden Ansprüche, **gekennzeichnet durch** eine Blende (3), die in der Symmetrieebene (A) angeordnet ist.

6. Endoskop (12, 13), umfassend ein Relaissystem (11) mit mindestens einer Relaisoptik (1) nach einem der vorhergehenden Ansprüche.

7. Endoskop (12, 13) nach Anspruch 6, umfassend einen starren Endoskopschaft, in dem das Relaissystem (11) angeordnet ist.

8. Endoskop (12, 13) nach Anspruch 6 oder 7, **dadurch gekennzeichnet, dass** das Relaissystem (11) mindestens ein Relaismodul (10) umfasst, das mehrere längs der optischen Achse (O) hintereinander angeordnete Relaisoptiken (1) nach einem der Ansprüche 1 bis 5 enthält.

9. Endoskop (13) nach Anspruch 8, **dadurch gekennzeichnet, dass** das Relaissystem (11) zwei Relaismodule (10) umfasst, die eine stereoskopische Anordnung bilden.

## Claims

1. A relay optical system (1) for a rigid endoscope (12, 13), comprising two identically formed lens systems (2) which are arranged symmetrically to each other with respect to a plane of symmetry (A) that is perpendicular to the optical axis (O), wherein at least one lens of the respective lens system has an antireflection coating, **characterized in that** the lens systems (2) each comprise a first biconvex lens (4), a biconcave lens (5), a rod lens (6) having a convex lens surface (F5) facing the plane of symmetry (A) and a concave lens surface (F6) facing away from the plane of symmetry (A) and a second biconvex lens (7), in this order as viewed from the plane of symmetry (A), wherein the first biconvex lens (4) and/or the second biconvex lens (7) of the respective lens system (2) have the antireflection coating.

2. The relay optical system (1) according to claim 1, **characterized in that** the first biconvex lens (4), the biconcave lens (5), the rod lens (6) and/or the second biconvex lens (7) of the respective lens system (2) are cemented to each other.

3. The relay optical system (1) according to claim 1 or 2, **characterized in that** the rod lens (6) of the respective lens system (2) is made of crown glass.

4. The relay optical system (1) according to one of the preceding claims, **characterized in that** the first biconvex lens (4), the biconcave lens (5) and/or the second biconvex lens (7) of the respective lens system (2) are made of flint glass.

5. The relay optical system (1) according to one of the preceding claims, **characterized by** a stop (3) which is arranged in the plane of symmetry (A).

6. An endoscope (12, 13), comprising a relay system (11) with at least one relay optical system (1) according to one of the preceding claims.

7. The endoscope (12, 13) according to claim 6, comprising a rigid endoscope shaft in which the relay system (11) is arranged.

8. The endoscope (12, 13) according to claim 6 or 7, **characterized in that** the relay system (11) comprises at least one relay module (10) having several relay optical systems (1) according to one of the claims 1 to 5 arranged successively along the optical axis (O).

9. The endoscope (13) according to claim 8, **characterized in that** the relay optical system (11) comprises two relay modules (10) forming a stereoscopic arrangement.

## Revendications

1. Optique de relais (1) pour un endoscope rigide (12, 13) comprenant deux systèmes de lentilles de conception identique (2) disposés symétriquement l'un par rapport à l'autre par rapport à un plan de symétrie (A) perpendiculaire à l'axe optique (O), au moins une lentille du système de lentilles concerné présentant une couche anti-réfléchissante, **caractérisée en ce que** les systèmes de lentilles (2) contiennent chacun une première lentille biconvexe (4), une lentille biconcave (5), une lentille à tige (6) qui a une surface de lentille convexe (F5) dirigée vers le plan de symétrie (A) et une surface de lentille concave (F6) opposée au plan de symétrie (A), et une deuxième lentille biconvexe (7) vue dans cet ordre depuis le plan de symétrie (A), la première lentille biconvexe (4) et/ou la deuxième lentille biconvexe (7) du système de lentilles (2) respectif présentant la couche anti-réfléchissante.

2. Optique de relais (1) selon la revendication 1, **caractérisée en ce que** la première lentille biconvexe (4), la lentille biconcave (5), la lentille à tige (6) et/ou la deuxième lentille biconvexe (7) du système de lentilles (2) respectif, sont mastiquées ensemble.

3. Optique de relais (1) selon la revendication 1 ou la revendication 2, **caractérisée en ce que** la lentille à tige (6) du système de lentilles (2) respectif est constituée d'un verre crown.

4. Optique de relais (1) selon l'une quelconque des revendications précédentes, **caractérisée en ce que** la première lentille biconvexe (4), la lentille biconcave (5) et/ou la deuxième lentille biconvexe (7) du système de lentilles (2) respectif sont constituées d'un verre flint.

5. Optique de relais (1) selon l'une quelconque des revendications précédentes, **caractérisée par** un diaphragme (3) disposé sur le plan de symétrie (A).

6. Endoscope (12, 13) comprenant un système de relais (11) comprenant au moins une optique de relais (1) selon l'une quelconque des revendications précédentes.

7. Endoscope (12, 13) selon la revendication 6, comprenant une tige d'endoscope rigide dans laquelle est disposé le système de relais (11).

8. Endoscope (12, 13) selon la revendication 6 ou la revendication 7, **caractérisé en ce que** le système de relais (11) comprend au moins un module relais (10) qui contient plusieurs optiques de relais (1), disposées l'une derrière l'autre le long de l'axe optique (O) selon l'une quelconque des revendications 1 à 5.

9. Endoscope (13) selon la revendication 8, **caractérisé en ce que** le système de relais (11) comprend deux modules relais (10) qui forment un montage stéréoscopique.
